# EUROPÄISCHE PATENTSCHRIFT

(11) **EP 2 178 437 B1**
(45) Veröffentlichungstag und Bekanntmachung des Hinweises auf die Patenterteilung: **10.04.2013**
(21) Anmeldenummer: 08785650.6
(22) Anmeldetag: 20.08.2008
(51) Int. Cl.: A61B 5/145, A61M 1/36, A61M 5/168, A61M 25/00

(54) **VORRICHTUNG ZUR ÜBERWACHUNG EINES ZUGANGS ZU EINEM PATIENTEN**
DEVICE FOR MONITORING AN ACCESS TO A PATIENT
DISPOSITIF DE SURVEILLANCE D'UN ACCÈS À UN PATIENT

(30) Priorität: 22.08.2007 DE 102007039581
(43) Veröffentlichungstag der Anmeldung: 28.04.2010
(73) Patentinhaber: Fresenius Medical Care Deutschland GmbH, 61352 Bad Homburg v.d.H. (DE)
(72) Erfinder: HEIDE, Alexander, 65817 Eppstein (DE); KLEWINGHAUS, Jürgen, 61440 Oberursel (DE); LANGKAU, Wolfram, 61449 Steinbach (DE)
(74) Vertreter: Oppermann, Frank
(86) Internationale Anmeldenummer: PCT/EP2008/006841
(87) Internationale Veröffentlichungsnummer: WO 2009/024333

(56) Entgegenhaltungen:
- FR-A- 2 788 222
- US-A1- 2003 128 125

## Beschreibung

Die Erfindung bezieht sich auf eine Vorrichtung zur Überwachung eines Zugangs zu einem Patienten für eine Einrichtung, mit der über eine Nadel oder Kanüle dem Patienten eine Flüssigkeit zugeführt wird, insbesondere zur Überwachung des venösen Gefäßzugangs bei einer extrakorporalen Blutbehandlung, bei der über eine arterielle Schlauchleitung, die eine arterielle Nadel oder Kanüle aufweist, dem Patienten Blut entnommen und über eine venöse Schlauchleitung, die eine venöse Nadel oder Kanüle aufweist, dem Patienten Blut zugeführt wird. Darüber hinaus betrifft die Erfindung eine Blutbehandlungsvorrichtung mit einem extrakorporalen Blutkreislauf, die über eine Vorrichtung zur Überwachung des venösen Gefäßzugangs verfügt.

Auf dem Gebiet der Medizintechnik sind Einrichtungen bekannt, mit denen Flüssigkeiten einem Patienten entnommen und dem Patienten Flüssigkeiten zugeführt werden können. Dabei kann der Zugang zu dem Patienten mit einer Nadel oder Kanüle zum Punktieren von Gefäßen erfolgen. Während der Untersuchung oder Behandlung ist ein ordnungsgemäßer Zugang zu dem Patienten sicher zu stellen. Daher ist es erforderlich, den Patientenzugang zu überwachen.

Ein Anwendungsfall mit besonders hohen Anforderungen an die Sicherheit des Gefäßzugangs stellt die extrakorporale Blutbehandlung dar, bei der über eine arterielle Blutleitung, die eine arterielle Punktionsnadel oder -kanüle aufweist, dem Patienten Blut entnommen wird, das Blut durch einen Dialysator geleitet wird und über eine venöse Blutleitung, die eine venöse Punktionsnadel oder -kanüle aufweist, dem Patienten wieder zugeführt wird. Dabei besteht trotz regelmäßiger Überwachung des Patientenzugangs durch das Krankenhauspersonal grundsätzlich die Gefahr, dass die venöse Nadel oder Kanüle unbemerkt aus dem Blutgefäß des Patienten herausrutscht. Während ein Herausrutschen der arteriellen Kanüle mit einem Ansaugen von Luft in die arterielle Schlauchleitung verbunden ist, das zu einem Alarm und zur Unterbrechung der Behandlung führt, ist das Herausrutschen der venösen Kanüle und der damit gefürchtete Freifluss des Bluts in die Umgebung nicht ohne weiteres zu detektieren. Wenn das Herausrutschen der venösen Kanüle aber nicht sofort erkannt wird, kann der Patient verbluten.

Zur Lösung dieses Problems sind im Stand der Technik unterschiedliche Vorrichtungen bekannt. Einige dieser Vorrichtungen greifen auf standardmäßig in den Blutbehandlungsmaschinen vorhandene Sicherheitseinrichtungen zurück und lösen bei einem nicht ordnungsgemäßen Gefäßzugang eine sofortige Unterbrechung des extrakorporalen Blutkreislaufs aus. Die standardmäßig in den Behandlungsmaschinen vorhandenen Sicherheitseinrichtungen basieren im Allgemeinen auf einer Überwachung des Drucks im extrakorporalen Blutkreislauf. In der Praxis hat sich jedoch gezeigt, dass allein mit einer Überwachung des Drucks im extrakorporalen Blutkreislauf das Herausrutschen insbesondere der venösen Nadel oder Kanüle nicht mit der ausreichenden Sicherheit erkannt werden kann. Einige bekannte Sicherheitsvorrichtungen haben zwar eine ausreichende Sensitivität, sie reagieren aber sehr empfindlich auf Lageänderungen des Patienten, was oft zu Fehlalarmen führt. Nachteilig ist auch, dass sich die bestehenden Blutbehandlungsvorrichtungen nicht ohne weiteres mit den bekannten Überwachungseinrichtungen nachrüsten lassen, sondern die Nachrüstung einen aufwendigen und kostenintensiven Eingriff in die Behandlungsmaschinen erfordert.

Aus der US 2004/0254513 A1 ist eine extrakorporale Blutbehandlungsvorrichtung mit einem extrakorporalen Blutkreislauf bekannt, die über eine Überwachungsvorrichtung für den arteriellen und venösen Gefäßzugang verfügt. Die bekannte Überwachungsvorrichtung weist zwei Elektroden auf, von denen die eine an der arteriellen und die andere an der venösen Schlauchleitung angeordnet ist, um über zwei Messleitungen eine elektrische Verbindung zwischen der Flüssigkeit in der jeweiligen Schlauchleitung und einer Überwachungseinheit herzustellen. Die Überwachungseinheit misst die Impedanz zwischen den beiden Elektroden, wobei auf einen nicht ordnungsgemäßen Gefäßzugang geschlossen wird, wenn die Impedanz nicht innerhalb vorgegebener Grenzen liegt. Nachteilig ist, dass die Überwachung des Gefäßzugangs zusätzliche Elektroden und Messleitungen erforderlich macht.

Die WO 97/10013 beschreibt eine auf der Überwachung des Drucks im extrakorporalen Blutkreislauf beruhende Überwachungsvorrichtung, bei der sich die aufgrund des Herzschlags erzeugten und in den arteriellen und venösen Schlauchleitungen fortpflanzenden Druckwellen detektiert werden. Das Ausbleiben der Drucksignale wird als ein Indikator für einen fehlerhaften Gefäßzugang angesehen.

Die WO 2004/110528 A1 beschreibt eine Vorrichtung zur Überprüfung einer Infusionspumpe, die auf der Überwachung des von dem Gerät im Betrieb emittierten Schalls beruht, der als Körper- oder als Luftschall erfasst werden kann. Dabei wird davon ausgegangen, dass die Infusionspumpe bei ordnungsgemäßem Betrieb Schall erzeugt, der bestimmte Charakteristika hat. Auf einen fehlerhaften Betriebszustand wird dann geschlossen, wenn der von der Pumpe erzeugte Schall von diesen Charakteristika abweicht. Dadurch können insbesondere Antriebsfehler, die beispielsweise auf schadhafte Lager zurückzuführen sind, aber auch Verschlüsse des Infusionskatheters erkannt werden.

Aus der US-A-5 662 619 ist eine venöse Nadel für den venösen Patientenzugang bei einer extrakorporalen Blutbehandlung bekannt. Die venöse Nadel weist am distalen Ende einen schrägen Schliff auf. Zur Reduzierung von Turbulenzen beim Austritt des Bluts aus der Nadel wird vorgeschlagen, an der Nadel laterale Öffnungen vorzusehen und stromab der lateralen Öffnungen in das Lumen vorspringende Ansätze auszubilden, die entgegen der Strömungsrichtung zeigen.

Die US 2003/0128125 A1 beschreibt ein Überwachungssystem für eine extrakorporale Blutbehandlungsvorrichtung, das eine Alarmeinrichtung umfasst. Die Alarmeinrichtung sieht unterschiedliche Alarmsignale für verschiedene Betriebszustände vor, die mit der Alarmeinrichtung überwacht werden. Zur Überwachung des Patientenzugangs wird vorgeschlagen, mit einem akustischen Sensor die Infiltration von Luft zu erkennen, wobei die durch Luftblasen entstehenden Geräusche erfasst werden sollen.

Der Erfindung liegt die Aufgabe zu Grunde, eine Vorrichtung zu schaffen, die eine sichere Überwachung eines Zugangs zu einem Patienten, insbesondere des venösen Patientenzugangs bei einer extrakorporalen Blutbehandlung auf einfache Weise erlaubt.

Eine weitere Aufgabe der Erfindung liegt darin, eine extrakorporale Blutbehandlungsvorrichtung mit einer Überwachungsvorrichtung für den venösen Patientenzugang bereit zu stellen, die eine sichere Überwachung des Zugangs auf einfache Weise erlaubt.

Die Lösung dieser Aufgaben erfolgt erfindungsgemäß mit den Merkmalen der Patentansprüche 1 und 9. Vorteilhafte Ausführungsformen der Erfindung sind Gegenstand der Unteransprüche.

Die erfindungsgemäße Vorrichtung zur Überwachung eines Patientenzugangs beruhen auf der Verwendung einer besonderen Nadel oder Kanüle für den Patientenzugang. Die Nadel oder Kanüle ist derart ausgebildet, das infolge der durch die Nadel oder Kanüle strömenden Flüssigkeit Luft angesaugt wird, wenn sich die Nadel oder Kanüle außerhalb des Gefäßzugangs befindet. Beim Ansaugen von Luft außerhalb des Gefäßzugangs wird wiederum Schall erzeugt. Daher kann ein nicht ordnungsgemäßer Gefäßzugang mit hoher Sicherheit allein dadurch erkannt werden, dass die Umgebung auf das Auftreten von Schall überwacht wird.

Es hat sich gezeigt, dass schon das Ansaugen geringer Mengen von Luft zu einem deutlich hörbaren Geräusch führen kann, wenn sich die Nadel oder Kanüle nicht innerhalb des Gefäßzugangs befindet. Daher ist es möglich, ein Herausrutschen der Nadel oder Kanüle aus dem Gefäßzugang mit hoher Sicherheit zu erkennen.

Für die Auswertung des Signals ist es aber grundsätzlich nicht erforderlich, dass der Schall hörbar ist. Der Schall kann auch als Druck- oder Dichteschwankungen in einem elastischen Medium, d.h. Gasen, Flüssigkeiten, Festkörpern nachgewiesen werden, ohne dass der Schall hörbar ist. So kann auch eine Auswertung von Festkörperschwingungen, z.B. mittels einer Oberflächenabtastung mit einem Laser oder mittels Beschleunigungssensoren erfolgen, die beispielsweise am Blutschlauch angebracht werden können.

Die erfindungsgemäße Überwachungsvorrichtung zeichnet sich dadurch aus, dass sie einfach zu handhaben ist, kostengünstig herstellbar ist und jederzeit nachrüstbar ist. Insbesondere findet die erfindungsgemäße Überwachungsvorrichtung zur Überwachung des venösen Gefäßzugangs bei einer extrakorporalen Blutbehandlung Verwendung. Ein weiterer Einsatzbereich ist die Überwachung eines Patientenzugangs bei einer Infusionseinrichtung. In beiden Fällen wird von einer Nadel oder Kanüle für den Patientenzugang Gebrauch gemacht.

Die erfindungsgemäße Überwachungsvorrichtung sieht vor, dass die Nadel oder Kanüle eine Verengung des Querschnitts und eine Öffnung aufweist. Die Nadel oder Kanüle stellt also eine Art Venturi-Düse dar. Die Querschnittsverringerung führt zu einer Erhöhung des dynamischen Drucks und Verringerung des statischen Drucks an der Engstelle. Dadurch steigt im Bereich des eingeschnürten Abschnitts der Nadel oder Kanüle die Geschwindigkeit der durch die Nadel oder Kanüle strömenden Flüssigkeit, wobei ein Unterdruck entsteht. Infolge des Unterdrucks wird durch die Öffnung in der Nadel oder Kanüle Luft angesaugt, wodurch Schall erzeugt wird, wenn die Nadel oder Kanüle nicht ordnungsgemäß im Gefäß des Patienten sitzt.

Grundsätzlich sind nur eine Engstelle in der Nadel oder Kanüle sowie nur eine Öffnung ausreichend, um Schall zu erzeugen, der sich sicher nachweisen lässt. Es ist aber auch möglich, mehrere Engstellen oder Öffnungen vorzusehen.

Bei einer besonders bevorzugten Ausführungsform wird die Verengung des Nadel- oder Kanülenquerschnitts durch eine Einschnürung der Nadel oder Kanüle geschaffen. Es ist aber auch möglich, dass die Engstelle durch nach innen vorspringende Ansätze gebildet wird.

Bei einer besonders bevorzugten Ausführungsform der Nadel oder Kanüle ist die Öffnung eine Durchgansbohrung in der Einschnürung der Nadel oder Kanüle oder eine Durchgangsfräsung in der Wandung der Nadel oder Kanüle. Dadurch wird erreicht, dass Luft im Bereich des größten Unterdrucks angesaugt wird. Grundsätzlich ist es aber auch möglich, die Öffnung in Strömungsrichtung vor oder hinter der Engstelle anzuordnen.

Die erfindungsgemäße Überwachungsvorrichtung verfügt über eine Auswerteinheit zur Erfassung von Schall, die derart ausgebildet ist, dass beim Erfassen von Schall auf einen nicht ordnungsgemäßen Patientenzugang geschlossen wird.

Die Auswerteinheit der erfindungsgemäßen Überwachungsvorrichtung weist vorzugsweise einen Schallwandler zur Umwandlung von Schallsignalen in elektrische Signale auf, die sich in einfache Weise mit den bekannten elektronischen Schaltungen verarbeiten und auswerten lassen. Als Schallwandler kommen alle Wandler in Betracht, mit denen sich Schallsignale in elektrische Signale umwandeln lassen. Beispielsweise elektrodynamische, kapazitive, piezoelektrisch oder piezoresistiv arbeitende Aufnehmer.

Das durch die in die Nadel oder Kanüle einströmende Luft erzeugte Geräusch wird neben dem hörbaren, luftgetragenen Schall, über den Körperschall übertragen, der sich in der Flüssigkeit und den anschließenden Schlauchleitungen fortpflanzt.

Eine weitere besonders bevorzugte Ausführungsform sieht vor, den Körperschall zur Erkennung eines nicht ordnungsgemäßen Gefäßzugangs zu überwachen. Bei dieser Ausführungsform verfügt die Auswerteinheit über einen Körperschallwandler zur Umwandlung von Körperschall in elektrische Signale. Als Körperschallwandler können beispielsweise Piezo-Tonabnehmer oder Elektret-Kondensatormikrofone Verwendung finden.

Der Körperschallwandler ist vorzugsweise an der Schlauchleitung angeordnet, über die sich der Körperschall fortpflanzt. Die permanente Überwachung des Körperschallsignals erlaubt eine sehr kurze Reaktionszeit, da das Störsignal zeitgleich mit der Diskonnektion der Nadel oder Kanüle auftritt.

Anstelle eines Körperschallwandlers können aber auch ein Laser zur Oberflächenabtastung der venösen Schlauchleitung oder ein an der Schlauchleitung angebrachter Beschleunigungssensor vorgesehen sein, mit denen sich Druck- oder Dichteschwankungen erkennen lassen.

Die elektrischen Signale, die in Abhängigkeit von den Druck- oder Dichteschwankungen von dem Schallwandler oder dem Laser zur Oberflächenabtastung oder dem Beschleunigungssensor erzeugt werden, können grundsätzlich auf unterschiedliche Weise ausgewertet werden. Bei einer bevorzugten Ausführungsform wird die Amplitude des elektrischen Signals überwacht und mit einem vorgegebenen Referenzsignal verglichen. Die Auswerteinheit schließt dann auf einen nicht ordnungsgemäßen Patientenzugang, wenn das elektrische Signal größer als das Referenzsignal ist. Dadurch ist es möglich, das Ausgangssignal von Störsignalen sicher zu unterscheiden.

Es ist aber auch möglich, nicht nur die Amplitude des Signals auszuwerten, sondern auch die Frequenz des Signals bei der Auswertung zu berücksichtigen. So können typische Klangsequenzen des Signals ausgewertet werden, die auch erst aus dem Signal herausgefiltert werden können. Beispielsweise ist ein Vergleich mit einem Signalmuster möglich, das für das Einsaugen von Luftblasen charakteristisch ist. Die Signalverarbeitung kann mit den bekannten analogen oder digitalen Techniken erfolgen.

Die erfindungsgemäße Überwachungsvorrichtung weist vorzugsweise Mittel auf, die bei der Erkennung eines nicht ordnungsgemäßen Patientenzugangs einen akustischen und/oder optischen Alarm geben. Darüber hinaus kann bei der Erkennung eines nicht ordnungsgemäßen Patientenzugangs ein Signal für einen Eingriff in die Maschinensteuerung der Blutbehandlungsvorrichtung zur Unterbrechung der Blutbehandlung erzeugt werden. Beispielsweise kann die Blutpumpe angehalten und durch Schließen der venösen Schlauchklemme an der venösen Blutleitung des extrakorporalen Blutkreislaufs der venöse Blutfluss unterbrochen werden.

Im Folgenden wird ein Ausführungsbeispiel der Erfindung unter Bezugnahme auf die Zeichnungen näher erläutert.

Es zeigen:
- Fig. 1: die wesentlichen Komponenten einer extrakorporalen Blutbehandlungsvorrichtung zusammen mit der erfindungsgemäßen Vorrichtung zur Überwachung des Patientenzugangs in stark vereinfachter schematischer Darstellung,
- Fig. 2: die venöse Nadel oder Kanüle des extrakorporalen Blutkreislaufs der extrakorporalen Blutbehandlungsvorrichtung bei einem ordnungsgemäßen Zugang zu dem venösen Gefäß des Patienten,
- Fig. 3: die venöse Nadel oder Kanüle von Figur 2 in vergrößerter Darstellung,
- Fig. 4: das Ausgangssignal des Schallwandlers der Auswerteinheit der erfindungsgemäßen Überwachungsvorrichtung bei einem ordnungsgemäßen Gefäßzugang,
- Fig. 5: die venöse Nadel oder Kanüle, wenn der Gefäßzugang nicht ordnungsgemäß ist,
- Fig. 6: die Kanüle von Figur 5 in vergrößerter Darstellung und
- Fig. 7: das Ausgangssignal des Schallwandlers der Auswerteinheit bei einem nicht ordnungsgemäßen Gefäßzugang.

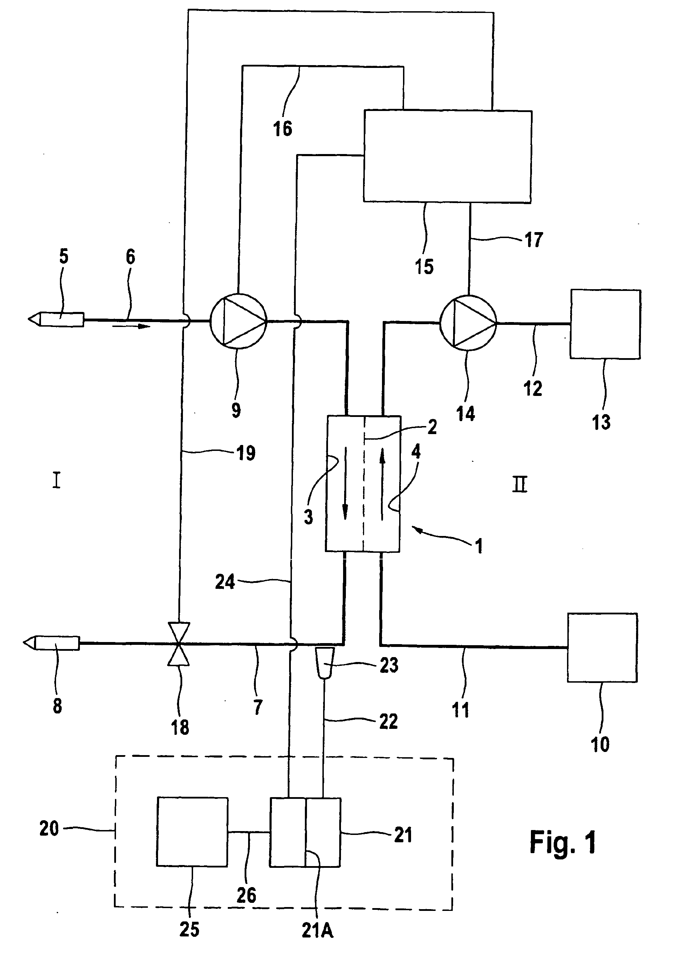

Fig. 1 zeigt die wesentlichen Komponenten einer extrakorporalen Blutbehandlungsvorrichtung, insbesondere einer Hämodialysevorrichtung, die über eine Vorrichtung zur Überwachung des venösen Gefäßzugangs verfügt. Die Hämodialysevorrichtung weist einen Dialysator 1 auf, der durch eine semipermeable Membran 2 in eine Blutkammer 3 und eine Dialysierflüssigkeitskammer 4 unterteilt ist.

An eine Arterie des Patienten ist mittels einer arteriellen Punktionsnadel oder -kanüle 5 eine arterielle Schlauchleitung 6 angeschlossen, die zum Einlass der Blutkammer 3 des Dialysators führt. Von dem Auslass der Blutkammer 3 des Dialysators 1 geht eine venöse Schlauchleitung 7 ab, die mittels einer venösen Punktionsnadel oder -kanüle 8 an einer Vene des Patienten angeschlossen ist. Die arterielle Schlauchleitung 6 ist in eine okkludierende Blutpumpe 9 eingelegt, die das Blut im extrakorporalen Blutkreislauf I fördert.

Der Dialysierflüssigkeitskreislauf II der Hämodialysevorrichtung umfasst eine Dialysierflüssigkeitsquelle 10, an der eine Dialysierflüssigkeitszuführleitung 11 angeschlossen ist, die zu dem Einlass der Dialysierflüssigkeitskammer 4 des Dialysators 1 führt. Von dem Auslass der Dialysierflüssigkeitskammer 4 des Dialysators 1 geht eine Dialysierflüssigkeitsabführleitung 12 ab, die zu einem Auslass 13 führt. In die Dialysierflüssigkeitsabführleitung 12 ist eine Dialysierflüssigkeitspumpe 14 zum Fördern der Dialysierflüssigkeit geschaltet.

Die Steuerung der Dialysevorrichtung übernimmt eine zentrale Steuereinheit 15, die über Steuerleitungen 16, 17 die Blut- und Dialysierflüssigkeitspumpe 9, 14 ansteuert.

Stromab der Blutkammer 3 des Dialysators 1 befindet sich an der venösen Schlauchleitung 7 eine elektro-magnetisch betätigbare Schlauchklemme 18, die über eine weitere Steuerleitung 19 von der zentralen Steuereinheit 15 geschlossen wird, wenn die venöse Punktionsnadel oder -kanüle aus dem Gefäßzugang herausrutschen sollte. Darüber hinaus stoppt die Steuereinheit 15 nach dem Herausrutschen der Kanüle die Blutpumpe 9.

Zur Überwachung des venösen Gefäßzugangs ist eine Überwachungseinrichtung 20 vorgesehen, die eine selbständige Einheit bildet oder Bestandteil der Dialysevorrichtung sein kann. Für den Fall, dass eine konventionelle Dialysevorrichtung mit einer Vorrichtung zur Überwachung des venösen Gefäßzugangs nachgerüstet werden soll, ist die Überwachungsvorrichtung 20 eine selbständige Einheit. Ansonsten ist die Überwachungseinrichtung vorzugsweise Bestandteil der Dialysevorrichtung, da die Dialysevorrichtung bereits über verschiedene Komponenten verfügt, von denen die Überwachungsvorrichtung Gebrauch machen kann. Hierzu gehören beispielsweise die zentrale Steuerung (Mikroprozessor) der Dialysevorrichtung und die Stromversorgung (Netzteil).

Die Überwachungsvorrichtung 20 verfügt über eine Auswerteinheit 21, die über eine Datenleitung 22 mit einem Schallwandler 23 verbunden ist, der Körperschall an der venösen Schlauchleitung 7 misst. Anstelle eines Körperschallwandlers kann aber auch ein Laser zur Oberflächenabtastung der venösen Schlauchleitung oder ein an der venösen Schlauchleitung angebrachter Beschleunigungssensor vorgesehen sein, mit denen Schwingungen erkannt werden können. Die Auswerteinheit 21 kommuniziert mit der zentralen Steuereinheit 15 der Dialysevorrichtung über eine weitere Datenleitung 24.

Die Überwachungsvorrichtung 20 weist eine Alarmeinheit 25 auf, die über eine Datenleitung 26 mit der Auswerteinheit 21 verbunden ist. Die Alarmeinheit 25, bei der es sich auch um die zentrale Alarmeinheit der Dialysevorrichtung handeln kann, erzeugt einen akustischen und/oder optischen Alarm, wenn die Überwachungsvorrichtung 20 einen nicht ordnungsgemäßen venösen Gefäßzugang erkennt. Darüber hinaus erzeugt die Auswerteinheit 21 bei einem nicht ordnungsgemäßen Gefäßzugang ein Steuersignal, das die zentrale Steuereinheit 15 der Dialysevorrichtung über die Steuerleitung 24 empfängt. Wenn die zentrale Steuereinheit 15 das Steuersignal der Auswerteinheit 21 empfängt, wird die Blutbehandlung unterbrochen, wobei die Steuereinheit 15 die venöse Schlauchklemme 18 schließt und die Blutpumpe 9 stoppt.

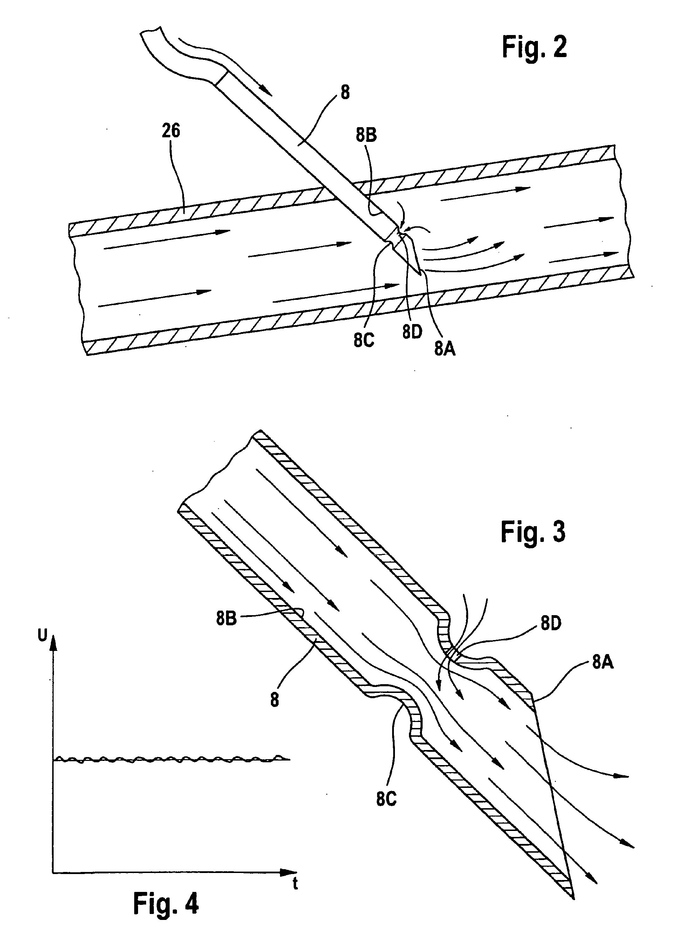

Die Figuren 2 und 3 zeigen die venöse Punktionsnadel oder -kanüle 8 bei einem ordnungsgemäßen Gefäßzugang. An der Nadel oder Kanüle ist die Schlauchleitung 7 angeschlossen, an der der Körperschallwandler 23 angebracht ist.

Bei der Nadel oder Kanüle handelt es sich grundsätzlich um eine konventionelle Punktionsnadel oder -kanüle, die aber am distalen Ende besonders ausgebildet ist.

Zum Punktieren des Gefäßes 26 weist die Nadel oder Kanüle am distalen Ende einen schrägen Schliff 8A auf. Bei einem ordnungsgemäßen Gefäßzugang, d.h. die Nadel oder Kanüle ist in das Gefäß 26 gestochen, strömt durch das Lumen 8B der Nadel oder Kanüle Blut aus der Blutkammer 3 des Dialysators 1 in das Gefäß 26 des Patienten, während Blut des Patienten gleichzeitig durch das Blutgefäß 26 strömt. Diese Blutströmung ist in den Figuren 2 und 3 mit Pfeilen gekennzeichnet.

Am distalen Ende weist die Nadel oder Kanüle 8 eine Verengung 8C auf, die durch eine Einschnürung mit einem düsenförmigen Querschnitt gebildet wird. Die Einschnürung 8C kann beispielsweise die Kontur einer Venturi-Düse beschreiben. Im Bereich der Einschnürung 8C ist der Strömungsquerschnitt im Verhältnis zum Durchmesser der Nadel oder Kanüle um ca. 10 bis 40 %, vorzugsweise 15 bis 25 % verringert. Innerhalb der Einschnürung 8C befindet sich in der Wandung der Nadel oder Kanüle eine Durchgangsbohrung 8D oder eine Durchgangsfräsung durch die Wandung der Nadel oder Kanüle. Da die Einschnürung den Strömungswiderstand erhöht, sollte sie nur so groß bemessen werden, dass die Funktion gewährleistet ist.

Bei einem Ausführungsbeispiel ist der Nadelaußendurchmesser 1,8 mm, der kleinste Außendurchmesser der Einschnürung 1,5 mm, die Länge der Einschnürung ist 2 mm und der Durchmesser der Durchgangsbohrung ist 0,3 mm. Das Verhältnis von der Einschnürung zu dem Durchmesser der Nadel ist 16 %.

Die Nadel kann beispielsweise hergestellt werden, in dem beispielsweise durch Kaltverformung eine umlaufende Rille in die Wandung eines dünnwandigen Röhrchens geprägt wird, so dass sich der Strömungsquerschnitt verjüngt. Durch Auswahl einer passenden Form für das Prägewerkzeugs kann eine geeignete Kontur geschaffen werden.

Die Durchgangsbohrung 8D sollte zusammen mit der Einschnürung 8C so nahe wie möglich am Beginn des Schliffs 8A der Nadel oder Kanüle liegen, so dass einerseits sichergestellt ist, dass bei ordnungsgemäßem Sitz der Nadel bzw. Kanüle im Blutgefäß des Patienten sich deren seitliche Öffnung stets in einem ausreichenden Sicherheitsabstand zum Blutgefäß befindet, so dass keine Luft angesaugt werden kann, und andererseits gewährleistet ist, dass beim Herausziehen der Nadel Luft erst dann angesaugt werden kann, wenn die Nadel bereits gefährlich weit herausgerutscht ist, so dass für den Patienten Lebensgefahr besteht. Bei dem Ausführungsbeispiels liegt die Bohrung 8D der Spitze der Nadel oder Kanüle gegenüber.

Aufgrund der Verringerung des Strömungsquerschnitts entsteht im Bereich der Einschnürung 8C ein Unterdruck, so dass aus dem Gefäß 26 Blut in die Nadel oder Kanüle angesaugt wird. Dieses Blut strömt dann wieder aus der Nadel oder Kanüle in das Gefäß zurück. Da die Bohrung 8D einen kleinen Durchmesser hat, ist die zusätzliche Blutströmung relativ klein oder tritt je nach den Strömungsverhältnissen, insbesondere aufgrund der gegenüber Luft viel größeren Viskosität von Blut, gar nicht auf. Diese Blutströmung ist in den Figuren 2 und 3 wieder durch Pfeile gekennzeichnet.

Fig. 4 zeigt schematisch das elektrische Ausgangssignal des Körperschallwandlers 23, das die Auswerteinheit 21 der Überwachungsvorrichtung 20 empfängt. Das elektrische Ausgangssignal hat einen Gleichanteil und einen Wechselanteil. Der Wechselanteil des Ausgangssignals hat eine nur sehr kleine auf Störsignale zurückzuführende Amplitude, da die Nadel oder Kanüle in dem Gefäß ordnungsgemäß sitzt (Fig. 2, Fig. 3).

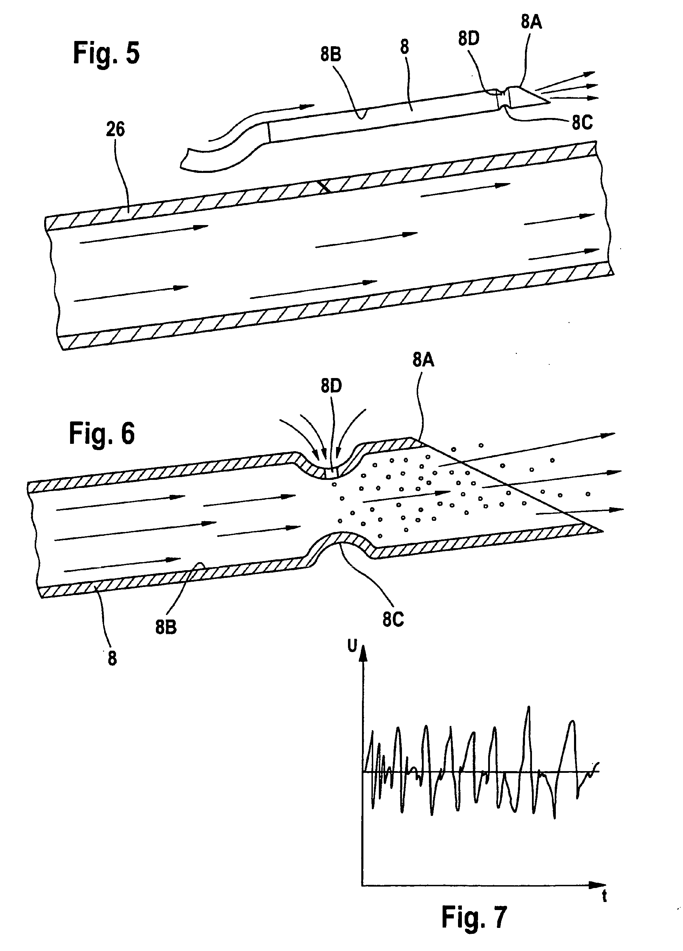

Die Figuren 5 und 6 zeigen den Fall, dass die Nadel oder Kanüle 8 aus dem venösen Gefäß 26 herausgerutscht ist. Da sich die Nadel oder Kanüle nicht mehr in dem Gefäß befindet, wird aufgrund des Unterdrucks Umgebungsluft angesaugt. Die angesaugte Umgebungsluft wird zusammen mit dem zuströmenden Blut wieder ausgestoßen. Dabei wird Schall erzeugt.

Das durch die einströmende Luft erzeugte Geräusch pflanzt sich neben dem hörbaren luftgetragenen Schall auch als Körperschall im Blut und der sich an die Nadel oder Kanüle anschließenden Blutleitung 7 fort. Der stromauf der Nadel oder Kanüle 8 an der venösen Schlauchleitung, 7 angebrachte Körperschallwandler 23 misst den Körperschall, der bei dem nicht ordnungsgemäßen Gefäßzugang auftritt.

Fig. 7 zeigt schematisch das elektrische Ausgangssignal des Körperschallwandlers 23. Deutlich ist zu erkennen, dass das Ausgangssignal zwar den gleichen Gleichanteil wie das Ausgangssignal bei einem ordnungsgemäßen Gefäßzugang (Fig. 4) hat, jedoch der Wechselanteil des Körperschallsignals eine relativ große Amplitude hat.

Die Auswerteinheit 21 der Überwachungsvorrichtung 20 weist eine Vergleichseinheit 21A zum Vergleichen der Amplitude des Wechselanteils des gemessenen Körperschallsignals mit einem Referenzsignal auf. Das Referenzsignal ist derart bemessen, dass es deutlich über dem Pegel der Störsignale, aber auch unter dem Pegel liegt, den der Wechselanteil des Körperschallsignals bei nicht ordnungsgemäßem Gefäßzugang hat.

Für die Erkennung des fehlerhaften Gefäßzugangs kann die Auswerteinheit über den Fachmann bekannte Einrichtungen verfügen, beispielsweise einen Gleichrichter zum Gleichrichten des Ausgangssignals und einen Komparator für den Vergleich mit dem Referenzsignal oder auch Filter etc.

Wenn der Wechselanteil des gemessenen Körperschallsignals größer als der Referenzpegel ist, wird auf einen fehlerhaften Gefäßzugang geschlossen, d.h. darauf geschlossen, dass die Nadel oder Kanüle aus dem Gefäß herausgerutscht ist. Die Auswerteinheit 21 erzeugt dann ein Alarmsignal, so dass die Alarmeinheit 25 einen akustischen und/oder optischen Alarm gibt und ein Steuersignal, so dass die zentrale Steuereinheit 15 der Dialysevorrichtung die venöse Schlauchklemme 18 schließt, die Blutpumpe 9 stoppt und die Behandlung unterbricht. Dadurch wird der gefürchtete Freifluss des Bluts verhindert.

Beim Herausrutschen der Nadel oder Kanüle aus dem Blutgefäß tritt unter bestimmten Strömungsbedingungen zunächst ein winziger Strahl Blut aus der seitlichen Bohrung aus, wobei Luft erst dann angesaugt wird, wenn der Schliff der Nadel bzw. Kanüle aus dem Blutgefäß vollständig herausgezogen ist, weil dann der patientenseitige Gegendruck entfällt und der Freifluss in die Umgebung auftritt. Es wird also erst dann Luft angesaugt, wenn die Nadel vollständig aus dem Blutgefäß herausgezogen ist.

Bei einer alternativen Ausführungsform wird das elektrische Signal des Körperschallwandlers 23 in der Auswerteinheit 21 der Überwachungsvorrichtung 20 mit einem charakteristischen Signalmuster verglichen, das für den Schall charakteristisch ist, der nach dem Herausrutschen der Nadel oder Kanüle auftritt. Wenn das charakteristische Signalmuster in dem elektrischen Signal des Körperschallwandlers 23 erkannt wird, schließt die Auswerteinheit 21 auf das Vorliegen eines nicht ordnungsgemäßen Patientenzugangs.

## Patentansprüche

1. Vorrichtung zur Überwachung eines Zugangs zu einem Patienten für eine Einrichtung, mit der über eine Nadel (5, 8) oder Kanüle dem Patienten eine Flüssigkeit zugeführt wird, insbesondere zur Überwachung des venösen Gefäßzugangs bei einer extrakorporalen Blutbehandlung (I), bei der über eine arterielle Schlauchleitung (6), die eine arterielle Nadel (5) oder Kanüle aufweist, dem Patienten Blut entnommen und über eine venöse Schlauchleitung (7), die eine venöse Nadel (8) oder Kanüle aufweist, dem Patienten Blut zugeführt wird, wobei
die Vorrichtung (20) zur Überwachung des Patientenzugangs eine Nadel oder Kanüle (8) aufweist, die derart ausgebildet ist, dass die Nadel oder Kanüle außerhalb des Gefäßzugangs infolge der durch die Nadel oder Kanüle strömenden Flüssigkeit Luft ansaugt, so dass beim Ansaugen der Luft Schall erzeugt wird, und die Vorrichtung (2) zur Überwachung des Patientenzugangs eine Auswerteinheit (21) zur Erfassung von Schall aufweist, die derart ausgebildet ist, dass beim Erfassen von Schall auf einen nicht ordnungsgemäßen Patientenzugang geschlossen wird,
**dadurch gekennzeichnet, dass**
die Nadel oder Kanüle (8) eine Verengung (8C) des Strömungsquerschnitts mit einer Öffnung (8D) zur Erzeugung eines Unterdrucks infolge der durch die Nadel oder Kanüle strömenden Flüssigkeit aufweist, so dass bei einem nicht ordnungsgemäßen Gefäßzugang durch die Öffnung Luft in die Nadel oder Kanüle angesaugt wird, so dass beim Ansaugen der Luft Schall erzeugt wird.

2. Vorrichtung nach Anspruch 1, **dadurch gekennzeichnet, dass** die Verengung der Nadel oder Kanüle (8) als eine Einschnürung (8C) ausgebildet ist, wobei die Öffnung (8D) eine Durchgangsbohrung oder eine Durchgangsfräsung durch die Wandung der Nadel oder Kanüle ist.

3. Vorrichtung nach Anspruch 1 oder 2, **dadurch gekennzeichnet, dass** die Auswerteinheit (21) einen Schallwandler (23) zur Umwandlung von Schallsignalen in elektrische Signale oder einen Laser zur Oberflächenabtastung oder einen Beschleunigungssensor aufweist, die ein elektrisches Signal erzeugen.

4. Vorrichtung nach Anspruch 3, **dadurch gekennzeichnet, dass** der Schallwandler als ein Körperschallwandler (23) zur Umwandlung von Körperschall in elektrische Signale ausgebildet ist.

5. Vorrichtung nach Anspruch 4, **dadurch gekennzeichnet, dass** der Körperschallwandler (23) an der venösen Schlauchleitung (7) zur Erfassung von sich über die Schlauchleitung fortpflanzendem Körperschall angeordnet ist.

6. Vorrichtung nach einem der Ansprüche 3 bis 5, **dadurch gekennzeichnet, dass** die Auswerteinheit (21) eine Vergleichseinheit (21A) zum Vergleichen der Amplitude des elektrischen Signals mit einem vorgegebenen Referenzsignal aufweist, wobei die Auswerteinheit (21) auf einen nicht ordnungsgemäßen Patientenzugang schließt, wenn das elektrische Signal größer als das Referenzsignal ist.

7. Vorrichtung nach einem der Ansprüche 3 bis 5, **dadurch gekennzeichnet, dass** die Auswerteinheit (21) eine Vergleichseinheit (21 A) zum Vergleichen des elektrischen Signals mit einem charakteristischen Signalmuster aufweist, wobei die Auswerteinheit (21) auf einen nicht ordnungsgemäßen Patientenzugang schließt, wenn das charakteristische Signalmuster in dem elektrischen Signal erkannt wird.

8. Vorrichtung nach einem der Ansprüche 1 bis 7, **dadurch gekennzeichnet, dass** die Vorrichtung zur Überwachung eines Gefäßzugangs Mittel (21, 25) aufweist, die bei der Erkennung eines nicht ordnungsgemäßen Patientenzugangs einen akustischen und/oder optischen Alarm geben und/oder ein Signal für einen Eingriff in die Maschinensteuerung der Blutbehandlungsvorrichtung zur Unterbrechung der Blutbehandlung erzeugen.

9. Blutbehandlungsvorrichtung mit einem extrakorporalen Blutkreislauf, der eine arterielle Schlauchleitung (6) mit einer arteriellen Nadel oder Kanüle (5) und eine venöse Schlauchleitung (7) mit einer venösen Nadel oder Kanüle (8) aufweist, und mit einer Vorrichtung (20) zur Überwachung des venösen Gefäßzugangs nach einem der Ansprüche 1 bis 8.

## Claims

1. Device for monitoring access to a patient for a process involving: the use of a needle (5, 8) or a cannula to administer a fluid to a patient, in particular to monitor vascular access during extra-corporeal blood treatment (I), in which an arterial tube (6), comprising an arterial needle (5) or a cannula, is used to withdraw blood from a patient, and the use of a venous tube (7), comprising a venous needle (8) or a cannula, is used to return blood to a patient, whereby
the device (20) for monitoring access to a patient comprises a needle or a cannula (8), which is shaped in such a way that, outside the vascular access, the needle or cannula draws in air because of the fluid flowing through the needle or the cannula, and a noise is generated as the air is drawn in and the device (2) to monitor the access to the patient comprises an evaluation unit (21) to evaluate the noise, is constructed in such a manner that while the noise is being evaluated, any incorrect access to the patient can be indicated,
**characterised in that**
the needle or cannula (8) comprises a narrowing (8C) in the flow cross-section with an opening (8D) to generate an underpressure as a result of the fluid flowing through the needle or the cannula, so that in the event of an incorrect vascular access through the opening, air is drawn into the needle or the cannula so that a noise is generated when the air is drawn in.

2. Device according to Claim 1 above, **characterised in that** the narrowing in the needle or the cannula (8) is in the form of a constriction (8C), whereby the opening (8D) is an aperture that has been drilled or cut through the wail of the needle or the cannula.

3. Device according to Claim 1 or Claim 2 above, **characterised in that**; the evaluation unit (21) comprises a sound converter (21) to convert noise signals into electrical signals, or a laser to scan the surface or an acceleration sensor, which can generate an electrical signal.

4. Device according to Claim 3 above **characterised in that** the sound converter is in the form of a structure-borne noise converter (23) that can convert structure-borne noise into electrical signals.

5. Device according to Claim 4 above **characterised in that** the structure-borne noise converter (23) is arranged at the venous tube (7) in order to calculate structure- borne noise propagated through the tube.

6. Device according to any of the preceding Claims 3 to 5, **characterised in that** the evaluation unit (21) comprises a comparative unit (21A) to compare the amplitude of the electrical signal with a predetermined reference signal, whereby the evaluation unit (21) indicates an incorrect access to the patient if the electrical signal is greater than the reference signal.

7. Device according to any of the preceding Claims 3 to 5, **characterised in that** the evaluation unit (21) comprises a comparative unit (21A) to compare the electrical signal with a characteristic signal pattern, whereby the evaluation unit (21) indicates an incorrect access to the patient if the characteristic signal pattern is recognised in the electrical signal.

8. Device according to any of the preceding Claims 1 to 7, **characterised in that** the device to monitor the vascular access comprises means (21, 25), by which, if an incorrect access to the patient is identified, an acoustic and/or a visual alarm and/or signal is generated indicating that an intervention is required in the machine control system of the blood treatment device in order to interrupt the blood treatment.

9. Blood treatment device with an extra-corporeal blood circulation system, which comprises an arterial tube (6) having an arterial needle or a cannula (5) and a venous tube (7) having a venous needle or cannula (8) and with a device (20) to monitor vascular access according to any of the preceding Claims t to 8.

## Revendications

1. Dispositif de surveillance d'un accès à un patient pour un appareil avec lequel, par le biais d'une aiguille (5, 8) ou d'une canule, un liquide est acheminé au patient, en particulier pour la surveillance de l'accès vasculaire veineux dans un traitement sanguin extracorporel (1), par lequel, par le biais d'un tuyau artériel (6) qui présente une aiguille ou une canule artérielle (5), du sang est prélevé du patient et, par le biais d'un tuyau veineux (7) qui présente une aiguille ou une canule veineuse (8), du sang est acheminé au patient,
le dispositif (20) pour la surveillance de l'accès au patient présentant une aiguille ou une canule (8) qui est conçue de telle sorte que l'aiguille ou la canule aspire à l'extérieur de l'accès vasculaire de l'air en conséquence de l'écoulement du liquide par l'aiguille ou la canule, de sorte que, lors de l'aspiration de l'air, un son soit généré, et le dispositif (2) de surveillance de accès au patient présentant une unité d'analyse (21) pour capter le son qui est ainsi formé, de sorte que lors du captage du son, on conclut à un accès non conforme au patient,
**caractérisé en ce que**
l'aiguille ou la canule (8) présente un rétrécissement (8C) de la section d'écoulement avec une ouverture (8D) pour générer une pression négative en conséquence de l'écoulement du liquide par l'aiguille ou la canule, de sorte que, lors d'un accès vasculaire non-conforme par l'ouverture, de l'air dans l'aiguille ou la canule soit aspiré de sorte que, lors de l'aspiration de l'air, un son soit généré.

2. Dispositif selon la revendication 1, **caractérisé en ce que** le rétrécissement de l'aiguille ou de la canule (8) est constitué comme un étranglement (8C), l'ouverture (8D) étant un trou débouchant ou un fraisage débouchant au travers de la paroi de l'aiguille ou de la canule.

3. Dispositif selon la revendication 1 ou 2, **caractérisé en ce que** l'unité d'analyse (21) présente un transducteur acoustique (23) pour la conversion des signaux sonores en signaux électriques ou un laser pour balayage de surface ou un capteur d'accélération qui génèrent un signal électrique.

4. Dispositif selon la revendication 3, **caractérisé en ce que** le transducteur acoustique est conçu comme un transducteur de bruit de structure (23) pour la conversion du bruit de structure en signaux électriques.

5. Dispositif selon la revendication 4, **caractérisé en ce que** le transducteur de bruit de structure (23) est disposé sur le tuyau veineux (7) pour capter le bruit de structure se propageant de celui-ci par le tuyau.

6. Dispositif selon l'une des revendications 3 à 5, **caractérisé en ce que** l'unité d'analyse (21) présente une unité de comparaison (21A) pour comparer les amplitudes de signal électrique avec un signal de référence prédéfini, l'unité d'analyse (21) concluant à un accès non-conforme au patient lorsque le signal électrique est supérieur au signal de référence.

7. Dispositif selon l'une des revendications 3 à 5, **caractérisé en ce que** l'unité d'analyse (21) présente une unité de comparaison (21A) pour comparer le signal électrique avec un modèle de signal caractéristique, l'unité d'analyse (21) concluant à un accès au patient non-conforme lorsque le modèle de signal caractéristique dans le signal électrique est reconnu.

8. Dispositif selon l'une des revendications 1 à 7, **caractérisé en ce que** le dispositif de surveillance d'un accès vasculaire présente des moyens (21, 25) qui émettent, lors de la reconnaissance d'un accès au patient non-conforme, une alarme acoustique et/ou optique et/ou génèrent un signal pour une intervention dans la commande de la machine du dispositif de traitement sanguin pour interrompre le traitement sanguin.

9. Système de traitement sanguin avec une circulation extracorporelle qui présente un tuyau artériel (6) avec une aiguille ou une canule artérielle (5) et un tuyau veineux (7) avec une aiguille ou une canule veineuse (8) et comportant un dispositif (20) de surveillance du tuyau veineux selon l'une des revendications 1 à 8.
